# EUROPÄISCHE PATENTSCHRIFT

(11) **EP 1 171 141 B1**
(45) Veröffentlichungstag und Bekanntmachung des Hinweises auf die Patenterteilung: **20.10.2004**
(21) Anmeldenummer: 00914012.0
(22) Anmeldetag: 12.04.2000
(51) Int. Cl.: A61K 35/78, A61P 29/00, A61P 35/00, A61P 25/02

(54) **ARZNEIMITTEL ZUR HEMMUNG DES NF-kB TRANSKRIPTIONSFAKTORS**
MEDICAMENT FOR INHIBITING THE NF-kB TRANSCRIPTION FACTOR
MEDICAMENT POUR INHIBER LE FACTEUR DE TRANSCRIPTION NF-kB

(30) Priorität: 14.04.1999 EP 99810308; 19.08.1999 CH 151999; 20.08.1999 EP 99810750
(43) Veröffentlichungstag der Anmeldung: 16.01.2002
(73) Patentinhaber: Max Zeller Söhne AG, 8590 Romanshorn (CH)
(72) Erfinder: HAMBURGER, Matthias, D-07743 Jena (DE); DANZ, Henning, D-07743 Jena (DE)
(74) Vertreter: Ritscher, Thomas, Dr.
(86) Internationale Anmeldenummer: PCT/CH2000/000214
(87) Internationale Veröffentlichungsnummer: WO 2000/061159

(56) Entgegenhaltungen:
- EP-A- 0 987 027
- WO-A-95/13807
- US-A- 5 178 865
- US-A- 5 665 393
- US-A- 5 837 257
- MURUGANANDAM A V ET AL: "EFFECT OF WRIGHTIA TINCTORIA ON THE BRAIN MONOAMINES AND METABOLITES IN RATS" BIOGENIC AMINES,GB,ORSAY, Bd. 14, Nr. 6, September 1998 (1998-09), Seiten 655-665, XP000881033 ISSN: 0168-8561
- MITSCHER L A ET AL: "ANTIMICROBIAL AGENTS FROM HIGHER PLANTS. NEW SYNTHESIS AND BIOACTIVITY OF TRYPTANTHRIN (INDOLO-(2,1-B)-QUINAZOLIN-6,12-DIONE) AND ITS ANALOGUES" HETEROCYCLES,XX,XX, Bd. 15, Nr. 2, 1981, Seiten 1017-1021, XP000893148 ISSN: 0385-5414

## Beschreibung

Die vorliegende Erfindung betrifft die Verwendung von bestimmten Indolo-[2, 1-b]chinazolin-6,12-dionen und von Stoffmischungen, insbesondere in der Form von Pflanzenextrakten, die solche Verbindungen enthalten, für die Herstellung von Arzneimitteln zur Hemmung des Transkriptionsfaktors NF-κB (kurz NF-κB) und/oder zur Hemmung der Cyclooxygenasen unter präferentieller Hemmung der Cyclooxygenase-2 (kurz COX-2).

Transkriptionsfaktoren sind Proteine, die das Ablesen der codierten Erbinformation durch eine spezifische Bindung an bestimmte DNA-Abschnitte induzieren können. Bei verschiedenen entzündlichen Prozessen spielt insbesondere der Transkriptionsfaktor NF-κB eine wichtige Rolle, da viele entzündungsfördernde Induktoren zu seiner Aktivierung führen. Der aktivierte Transkriptionsfaktor seinerseits führt zur Expression von verschiedenen Genprodukten, die ihrerseits die Synthese entzündungsfördernder Substanzen katalysieren. Von besonderer Bedeutung ist COX-2, die das Schlüsselenzym in der Synthese von entzündungsfördernden Prostaglandinen ist. Neben klassischen akut oder chronisch entzündlichen Prozessen ist NF-κB nach heutigem Stand des Wissens auch in weitere Krankheitsbilder wie z.B. Morbus Alzheimer impliziert.

In den letzten Jahren sind verschiedene Substanzen mit NF-κB-hemmender Wirkung beschrieben worden. Dazu gehören die Curcuminoide und Kaffeesäurederivate, Acetylsalicylsäure und Natriumsalicylat, das Alkaloid Tetrandrin sowie einige Sesquiterpenlactone. Die Hemmwirkung der Curcuminoide und der Karfeesäurederivate ist aber schwach und scheint nur durch die Radikalfänger-Eigenschaften der Verbindungen zustande zu kommen. Die Hemmwirkung der Salicylate ist ebenfalls schwach. Die NF-κB inhibierende Wirkung der Sesquiterpenlactone ist mit der Anwesenheit einer exocyclischen α-ständigen Methylengruppe an einem γ-Lactonring verknüpft. Die Methylengruppe geht aufgrund ihrer Reaktivität leicht kovalente Bindungen mit nukleophilen Gruppen (z.B. SH-Gruppen von Cystein) ein.

Wirkstoffe mit inhibierender Wirkung auf NF-κB bieten jedoch einen neuartigen Ansatz zur Beeinflussung des Entzündungsgeschehens und könnten zur Hemmung akuter und chronischer entzündlicher Prozesse, als Antirheumatika sowie zur Prävention und Behandlung von neurodegenerativen Erkrankungen eingesetzt werden.

Die Cyclooxygenasen zählen ihrerseits zu den Schlüsselenzymen in der Biosynthese der Eicosanoide und katalysieren die Umsetzung von Arachidonsäure zu den kurzlebigen Prostaglandinen G und H, die durch spontanen Zerfall und weitere enzymatische Reaktionen in eine Vielzahl von physiologisch wirksamen Prostanoiden umgesetzt werden. Eicosanoide sind parakrine Hormone, die sich von C₂₀-Fettsäuren (Arachidonsäure) ableiten. Wichtige acyclische Eicosanoide sind die Leukotriene und Lipoxine; cyclische Derivate sind die Prostaglandine und Thromboxane. Unter dem Oberbegriff "Prostanoide" werden allgemein Verbindungen zusammengefasst, die sich von PGH₂ (Prostaglandin H₂) ableiten, das seinerseits durch Cyclooxygenase aus Arachidonsäure gebildet wird. Die Eicosanoide haben vielerlei biologische Wirkungen, deren physiologische Basis wegen der Vielzahl von Strukturen und den teilweise gewebeabhängigen Wirkungen noch nicht vollständig geklärt sind.

Die Prostaglandine und Thromboxane spielen als Gewebshormone in einer Vielzahl von physiologischen und pathologischen Prozessen eine Rolle, unter anderem als Entzündungsmediatoren. Eine Hemmung der Cyclooxygenasen führt deshalb zur Reduktion pro-inflammatorischer wirkender Prostanoide. Dieser Mechanismus ist bei den nicht-steroidalen Antirheumatika (NSAIDs) für die anti-inflammatorischen und analgetischen Wirkungen, aber auch für zahlreiche Nebenwirkungen auf den Magen-Darm-Trakt sowie die Nieren und für die Blutgerinnung verantwortlich. Die Suche nach verbesserten Wirkstoffen geht daher weiter.

Die Bezeichnung "Cyclooxygenase" umfasst nach gegenwärtigem Wissen zwei Isozyme: Cyclooxygenase-1, die konstitutiv in vielen Zellen exprimiert wird und für die Synthese von Prostaglandinen und anderen Mediatoren sorgt, die in verschiedenen physiologischen Prozessen eine Rolle spielen, u.a. für die Aufrechterhaltung einer intakten Magenschleimhaut und einer normalen Nierenfunktion sowie der Aggregation der Blutplättchen; Cyclooxygenase-2 hingegen kommt in den verschiedenen Geweben nicht oder nur in geringerem Masse konstitutiv vor, wird aber im Zuge von entzündlichen Prozessen verstärkt exprimiert, was zur Bezeichnung von COX-2 als induzierbare Cyclooxygenase geführt hat.

Nach heutigem Wissen hat COX-2 physiologische Funktionen in der Niere, im Gehirn/Rückenmark und im weiblichen Reproduktionssystem, pathologische Funktionen in Entzündungsprozessen, bei Schmerz, Morbus Alzheimer und Krebs.

Die Wirkung der NSAIDs als Antirheumatika bzw. anti-inflammatorische Mittel beruht mindestens zum Teil auf einer allgemeinen, d.h. nicht-selektiven Hemmung beider Isozyme der Cyclooxygenase. Unerwünschte Nebenwirkungen, insbesondere Erosion der Magenschleimhaut und Beeinflussung der Blutgerinnung, sind im wesentlichen der Hemmung von Cyclooxygenase-1 und der damit zusammenhängenden Hemmung der Synthese physiologisch wichtiger Prostaglandine zuzuschreiben.

Von Stoffen mit selektiver, und zwar präferentieller Hemmwirkung auf die induzierbare Cyclooxygenase, d.h. COX-2, wären somit bei vergleichbarer Wirkung als Antiphlogistika bzw. Antirheumatika geringere Nebenwirkungen zu erwarten, weil mit diesen Stoffen die für eine Homöostase wichtigen Eicosanoide nicht oder nur in vermindertem Masse beeinflusst würden. Als "präferentielle Hemmung" wird im vorliegenden Zusammenhang eine relativ stärkere Hemmung verstanden, wobei sich diese Stärke in physiologisch signifikanter Weise äussert.

Eine erste Aufgabe der Erfindung ist es, neue Arzneimittel zur NF-κB-Hemmung und/oder zur direkten oder indirekten Hemmung der Cyclooxygenasen mit Präferenz für die Cyclooxygenase-2 zu bieten.

Es wurde gefunden, dass sich diese Aufgabe durch das natürlich vorkommende, aber auch synthetisch zugängliche und z. B. aus WO 95/13807 als Mittel zur Bekämpfung von Mycobacterium tuberculosis beschriebene Tryptanthrin (Formel I, in der R₁ bis R₈ Wasserstoffatome darstellen; systematische Bezeichnung Indolo[2,1-b]chinazolin-6,12-dion) und geeignet substituierte Derivate dieser Verbindung sowie geeignete Salze und Stoffmischungen lösen lässt, welche diese Verbindungen enthalten.

Die Erfindung betrifft daher Indolo[2,1-b]chinazolin-6,12-dione, d.h. Verbindungen der Formel (I) 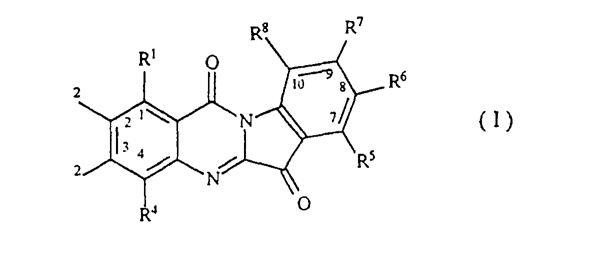 in der R¹ bis und mit R⁸ unabhängig voneinander gewählt sind aus Wasserstoffatomen und pharmazeutisch akzeptablen Substituenten, sowie die pharmazeutisch akzeptablen Salze der Verbindungen der Formel (I) zur Verwendung für die Herstellung von Arzneimitteln gemäss den Ansprüchen 1 - 4.

Es wird vermutet, dass die Hemmwirkung auf den Transkriptionsfaktor NF-κB mit einer Hemmung der Cyclooxygenasen unter präferentieller Hemmung der Cyclooxygenase-2 zusammenhängt. Wie weiter unten eingehender erläutert, zeigen bevorzugte erfindungsgemäss verwendete Stoffmischungen, nämlich bestimmte Pflanzenextrakte, zusätzlich zu der oben genannten und offensichtlich durch die darin enthaltenen Wirkstoffe der Formel (I) bedingten Hemmung des Transkriptionsfaktors NF-κB sowie der Hemmung der Cyclooxygenasen unter präferentieller Hemmung der Cyclooxygenase-2 auch eine Hemmung der Freisetzung von Histamin und/oder Serotonin und können daher auch zur Behandlung von Leiden verwendet werden können, die mit dieser Freisetzung zusammenhängen.

Serotonin (5-Hydroxytryptamin) und Histamin gehören zur Gruppe der biogenen Amine, die aus Vorläufersubstanzen (5- Hydroxytryptophan bzw. Histidin) durch Decarboxylierung gebildet werden. Beide Substanzen sind Mediatoren, (endogene Wirkstoffe), die durch unterschiedliche Stimuli aus Geweben oder Neubildungen im Blut freigesetzt werden können. Pathologische Bedeutung haben Histamin und Serotonin bei Entzündungsprozessen, Allergien und Schockzuständen. Darüber hinaus bewirken sie in Abhängigkeit vom Kreislaufabschnitt Vasodilatation oder Konstriktion, erhöhen die Gefässpermeabilität, Konstriktion der Uterus- und Brochialmuskulatur, dienen im Zentralnervensystem als Neurotransmitter und bewirken im Fall des Serotonins eine verstärkte Thrombozytenaggregation.

Bei verschiedenen Entzündungs- und allergischen Prozessen kommt es zu einer verstärkten Freisetzung von Histamin aus Mastzellen, vor allem bei der Allergie vom Typ I (Frühtyp), die sich in anaphylaktischem Schock. Urtikaria, Asthma, Rhinitis oder Konjunktivitis manifestieren kann. Beim Menschen dienen als Antikörper zellfixierte IgE-Gobuline, die (vereinfacht dargestellt) durch (bivalente) Antigene bruckenartig verknüpft werden und dadurch die Gefässpermeabilität der Mastzellen für Histamin erhöhen. Die Folge ist eine verstärkte Freisetzung an Histamin mit den oben beschriebenen pharmakologischen Wirkungen.

Histaminrezeptoren gehören zur Familie der G-Protein-gekoppelten Rezeptoren und lassen sich in verschiedene Subtypen (H₁, H₂, H₃) unterteilen. Sogenannte H₁-Antihistaminika werden hauptsächlich bei allergischen Prozessen und ferner auch als Antiemetika oder Hypnotica verwendet.

Serotonin kommt beim Menschen hauptsächlich in den Enterochrom-affinen Zellen der Darmmucosa, in den Thrombozyten und im Zentralnervensystem vor. Bisher sind schon zahlreiche Serotonin-Rezeptoren, sogenannte 5-HT-Rezeptoren (5-HT₁ bis 5HT₄ und weitere Subtypen), identifiziert worden.

Nach gegenwärtigem Stand des Wissens spielen 5-HT-Rezeptroen (5-HT_{2B}) im Bereich des trigemino-vaskulären Systems der Hirnhäute beim Krankheitsbild der Migräne eine besonders wichtige Rolle.

Eine selektive Hemmung der einzelnen Rezeptoren bewirkt in der Regel auch eine selektive pharmakologische Wirkung der entsprechenden Antagonisten. 5-HT-Rezeptor-Antagonisten werden als Psychopharmaka, Antihypertensiva und Antiemetika verwendet.

Aus Gewebe freigesetztes Serotonin und Histamin ist weiterhin auch in der Lage, die sogenannten Nozizeptoren, d.h. Schmerzrezeptoren, zu stimulieren. Eine Hemmung der Freisetzung dieser Mediatoren aus Mastzellen und Geweben bietet deshalb einen rationalen Ansatz für die Entwicklung von antiinflammatorisch und analgetisch wirkenden Substanzen.

Der genau physiologische Zusammenhang der verschiedenen, oben erläuterten Hemmwirkungen bzw. die Erklärung dieses Zusammenhangs ist noch nicht gesichert. Gesichert hingegen ist die der vorliegenden Erfindung zugrunde liegende Lehre, nämlich dass Verbindungen der Formel (I) und insbesondere die hier beschriebenen Stoffmischungen mit solcher Verbindungen in Form von Extrakten aus Isatis tinctoria die genannten Hemmwirkungen besitzen und sich daher zur Herstellung entsprechender Arzneimittel bzw. zur wirksamen Behandlung von chronischen oder akuten Zuständen eignen, die sich durch die genannten Hemmwirkungen positiv beeinflussen lassen.

Tryptanthrin (Couropitine A, Indolo[2,1-b]chinazolin-6,12-dion; Formel I, R¹ bis und mit R⁸ = H) ist ein Alkaloid, welches schon seit mehreren Jahrzehnten bekannt ist und bis heute in einigen Pflanzen (Strobilanthus cusia, Polygonum tinctorium, Isatis tinctoria, Courcoupita guianensis, Wrightia tinctoria, Calanthe spec.) und Mikroorganismen (Candida lipolytica) nachgewiesen und aus ihnen isoliert werden konnte. Das natürlich vorkommende oder das synthetisch erhaltene Tryptanthrin kann erfindungsgemäss als solches oder als Ausgangsstoff für die Herstellung von pharmazeutisch akzeptablen Derivaten bzw. Salzen verwendet werden.

Tryptanthrin und seiner Derivate wurden schon auf antimikrobielle Aktivität geprüft und wie oben erwähnt zur Inhibierung von Mycobacterium tuberculosis vorgeschlagen.

Die Wirkung dieser Stoffe auf die Hemmung des Transkriptionsfaktors NF-κB und/oder der Cyclooxygenasen unter präferentieller Hemmung der Cyclooxygenase 2 ist jedoch nach Wissen der Anmelderin bisher nicht bekannt geworden und war auf Grund der bekannten Eigenschaften auch nicht zu erwarten. Die gilt gleichermassen für die hier beschriebenen Stoffmischungen bzw. Extrakte, welche diese Hemmwirkungen zeigen und überdies auch ein Hemmwirkung auf die Freisetzung von Histamin und/oder Serotonin haben.

Die erfindungsgemäss eingesetzten Verbindungen der obigen Formel (I) können gemäss einer bevorzugten Ausführungsform in Form des auch natürlich vorkommenden Stoffes Tryptanthrin (Formel I, alle R₁ bis R₈ sind Wasserstoffatome), gewünschtenfalls in Form eines pharmazeutisch akzeptablen Salzes, verwendet werden.

Eine oder mehrere von R₁ bis R₈ können pharmazeutisch akzeptable Substituenten darstellen, die vorzugsweise aus folgenden Gruppen gewählt sind: Halogen (F, Cl, Br), C₁-C₁₀-Alkyl (Methyl bis Decyl, linear und verzweigt, gegebenenfalls durch Hydroxyl und/oder Halogen substituiert und/oder mit einer durch ein oder mehrere Sauerstoffatome unterbrochenen Kette), C₅-C₆-Cycloalkyl, gegebenenfalls ringsubstituierte Heterozyklen mit 6 bis 10 C-Atomen, Amino, Imino, Nitro, C₁-C₁₀-Alkylsulfonyl, C₆-C₁₀-Aryl, Phenyl, Arylalkyl (wobei die Alkyl- und Arylgruppen der obigen Definition entsprechen), Arylalkylaryl (wobei die Alkyl- und Arylgruppen der obigen Definition entsprechen), C₆-C₁₀-Aryloxy, C₆-C₁₀-Arylamino, Acylamino mit 1 - 10 C-Atomen in der Acylgruppe, Acyloxyamino mit 1 - 10 C-Atomen in der Acylgruppe, Alkylaminoacylamino mit insgesamt 1 - 10 C-Atomen, C₁-C₁₀-Alkylaminosulfonylamino, C₁-C₁₀-Alkylamino, C₁-C₁₀-Alkenylamino, C₁-C₁₀-Dialkylamino, C₁-C₁₀ Alkoxyalkylamino, Cyano, Formyl, COOR₁₁ , wobei R₁₁ Wasserstoff, C₁-C₁₀- Alkyl, C₆-C₁₀-Aryl, Heterozyklen mit insgesamt maximal 12 C-Atomen oder Mono- oder Disaccharid ist, und COONR₁₂R₁₃ , wobei R₁₂ und R₁₃ unabhängig voneinander die oben genannten Substituenten oder Aminosäure oder Peptid bedeuten können.

Pharmazeutisch akzeptable Salze von Verbindungen der Formel (I) sind Salze mit anorganischen oder organischen Säuren, wie typisch Salzsäure, Schwefelsäure, Phosphorsäure, Citronensäure, Milchsäure und dergleichen pharmazeutisch akzeptablen Säuren.

Die Indolo[2,1-b]chinazolin-6,12-dione der Formel (I) umschreiben eine neue Klasse von anti-inflammatorischen Wirkstoffen mit einem speziellen dualen Wirkmechanismus, nämlich auf der Ebene der Transkription und auf derjenigen der Eicosanoid-Synthese.

Auswählte Indolo[2,1-b]chinazolin-6,12-dione, wie z.B. bevorzugte Verbindungen der Formel (I), bei der alle R₁ bis R₈ Wasserstoffatome sind, und bevorzugte Verbindungen der Formel (I), in denen R₁,R₂ = R₄,R₅ = R₇,R₈ = H, R₃ ein Stickstoff enthaltender Substituent, wie 4-M, und R₆ Halogen, insbesondere Cl ist, bieten aussichtsreiche Mittel zur Behandlung von Zuständen, deren Zusammenhang mit der Aktivierung von NF-κB und/oder der Induktion der Bildung von COX-2 bereits bekannt ist, vermutet wird oder noch festzustellen ist, insbesondere als antiphlogistische bzw. antirheumatische Mittel, zur präventiven oder adjuvanten Behandlung von Morbus Alzheimer und von Krebserkrankungen sowie zur präventiven oder beziehungsweise adjunktiven Dauermedikation bei Altersbeschwerden.

Zu den bevorzugten Verbindungen der Formel (I) gehören ferner die Verbindungen, die bei den in den nachfolgenden Beispielen beschriebenen Prüfung NF-κB-Werte bei höchstens etwa 10, insbesondere höchstens etwa 5 µM/Liter und im COX-2-Testsystem IC₅₀-Werte von höchstens etwa 1, insbesondere höchstens etwa 0,05 µM/Liter ergeben, wobei die COX-1 Werte mindestens etwa doppelt so hoch sind, wie die COX-2-Werte, vorzugsweise mindestens etwa zehn mal höher.

Die Verbindungen der Formel (I) bzw. deren pharmazeutisch akzeptable Salze können mit den zur Herstellung von flüssigen oder festen Arzneimittelzubereitungen üblichen Adjuvantien, Streckmitteln, Trägern, Tablettierungshilfsmitteln, Aromastoffen und dergleichen in üblicher Weise verarbeitet werden. Typische Dosierungen werden im Bereich von 1 - 100 mg /Tag liegen, doch ist die Obergrenze voraussichtlich nicht kritisch, da bisher in den relevanten Bereichen keine Toxizität für Warmblüter festgestellt werden konnte.

Bevorzugte Stoffmischungen zur Verwendung gemäss der Erfindung sind Extrakte von Pflanzen, vorzugsweise Extrakte von Isatis tinctoria, die mindestens einen Wirkstoff der Formel (I), insbesondere Tryptanthrin, enthalten. Derartige Extrakte können bereits als solche, d.h. ohne Isolation von Wirkstoff, und einfach nach Wirkstoffkontrolle durch Abpacken, in die Form von Arzneimitteln zur Behandlung von Leiden gebracht werden können, welche durch die von Wirkstoff der Formel (I) verursachten Hemmwirkungen günstig beeinflusst werden.

Isatis tinctoria (Färberwaid), aus der ein bevorzugter Extrakt der eben genannten Art erhalten werden kann, ist eine bekannte Pflanze, deren Blätter (Isatidis folium) als Blaufärbemittel verwendet wurden, weil sie Glycoside von Indican bzw. Isatan B enthalten, die durch Hydrolyse in Indoxyl umgewandelt werden können, das seinerseits durch Oxidation den auch synthetisch zugänglichen Farbstoff Indigo bildet.

Die Herstellung des Farbstoffes Indigo aus Isatis tinctoria (Färberwaid) ist seit langem bekannt. Die Herstellung eines technisch reinen Farbstoffes ist beispielsweise in DE-A-4 211 719 beschrieben. Bei diesem Herstellungsverfahren werden jedoch die anderen Inhaltsstoffe der Pflanze, insbesondere weitere Tryptophanverbindungen, Anthranilsäurederivate, Senfölglycoside, Flavonoide, Terpene, Lignane als Abfallstoffe angesehen und nicht als Produkt gewonnen.

Ferner gibt es eine Vielzahl von Schriften im Zusammenhang mit der traditionellen chinesischen Medizin, die Kombinationspräparate aus verschiedenen Arzneimittelpflanzen mit sehr unterschiedlichen Indikationen und fehlenden oder kaum überprüfbaren Wirkungsangaben betreffen und gelegentlich auch die Pflanze Isatis tinctoria erwähnen, die schon seit Plinius als blutstillendes Mittel bekannt ist.

So ist z.B. in US-A-5 665 393 ein Kombinationspräparat aus acht verschiedenen Arzneipflanzen der Traditionellen Chinesischen Medizin beschrieben, wobei als neu eben diese Kombination zur Behandlung von Prostatakarzinom vorgeschlagen wird. Das Kombinationspräparat enthält neben Panax (pseudo-Ginseng), Ganoderma, Dentranthema, Glycyrrhiza, Scutellaria, Rabdiosa und Serenoa auch Isatis. Zum Nachweis einer pharmazeutischen Wirkung dienen die Hemmwirkung auf eine bestimmte Prostatakarzinomzellen und zwei Einzelfallbeschreibungen, bei welchen das Extraktgemisch zusammen mit einer schulmedizinischen Chemotherapie angewendet wurde.

Auch US-A-5 837 257 beruht auf der Heilstoffkunde der Traditionellen Chinesischen Medizin und betrifft ein Verfahren zur Behandlung von Infektionen mit Hepatitis-, Leukämie- und HIV-Viren, wobei mindestens eine der Heilpflanzen Solanum, Lespedeza, Senecio und Ligustrum, gegebenenfalls in Kombination mit vierzehn anderen Heilpflanzen, unter denen auch Isatis sp. genannt sind. Der Wirkungsnachweis stützt sich auf einen in vitro Nachweis einer anti-HIV Wirkung von wässerigen Extrakten und durch Anwendung bei Hepatitispatienten. Die antivirale Wirkung wird keiner einzelnen Pflanze sondern deren zahlreichen Kombinationen zugeschrieben:

Weitere Beispiele dieser Art finden sich in der chinesisch Patentliteratur: CN19930117037 ( Referenz XP 002116584 der Patentdatenbank WPI), CN 19930100926 (XP 002116585 - WPI), CN19930109189 (XP 002116586 - WPI), CN19980116929 (XP 002116587 - WPI), CN19980113551 (XP 002116588 - WPI) und der ebenfalls auf der chinesischen Arzneistoffiradition beruhenden JP19870331601 (XP 002116589 - WPI), die eine medizinische Zahnpaste zur Behandlung von Blutungen und Entzündungen des Mundes und mit Wirkung als Haartonikum beschreibt.

Der oben dargelegte Stand der Technik zur Verwendung von Isatis tinctoria kann dahingehend zusammengefasst werden, dass es sich dabei stets um Vielkomponentengemische handelt, die Isatis tinctoria als eine von mehreren pflanzlichen Komponenten enthalten und mit einer Ausnahme keine spezifizierten pharmazeutischen Wirkungen von Isatis tinctoria, geschweige denn speziell wirksame Inhaltsstoffe dieser Pflanzen, offenbaren. Die geltend gemachten Wirkungen werden vielmehr stets den besonderen Kombinationen von Heilmittelpflanzen zugeschrieben.

Extrakte von Isatis tinctoria stellen wie bereits kurz erwähnt eine bevorzugte Ausführungsform eines erfindungsgemäss verwendbaren Stoffgemische dar und sind wie auch Extrakte aus anderen, mindestens den Wirkstoff Tryptanthrin enthaltenden Pflanzen nach an sich bekannten Extraktionsmethoden erhältlich.

Zur Gewinnung von erfindungsgemäss verwendbaren Extrakten sind verschiedene Methoden geeignet. Dabei wird insbesondere die Hochdruckextraktion mit flüssigen (unterkritischen) oder überkritischen Gasen, z.B. Niederalkanen, wie z.B. Propan, Butan, oder (bevorzugt) CO₂ in reinem Zustand oder in Mischung mit unter Normalbedingungen flüssigen organischen Lösungsmitteln bevorzugt.

Allgemein können erfindungsgemäss verwendbare Extrakte mit Hilfe von Lösungsmitteln bzw. Lösungsmittelgemischen erhalten werden, die in der Lage sind, lipophile bis mässig polare Inhaltsstoffe des zu extrahierenden Pflanzenmaterials, wie insbesondere Isatis tinctoria, zu extrahieren. Ausser der genannten Hochdruckextraktion kann auch mit normalerweise flüssigen Lösungsmitteln, z.B. Alkoholen, wie Ethanol, Halogenkohlenwasserstoffen, z.B. Dichlormethan, und Petrolether extrahiert werden.

Erfindungsgemäss verwendbarer Extrakt aus Isatis tinctoria wird in der Regel aus den Blättern gewonnen, kann aber auch aus allen anderen Teilen der Pflanze, insbesondere dem Wurzel material, erhalten werden. Solche Extrakte enthalten neben Tryptanthrin eine Vielzahl unterschiedlicher chemischer Stoffe, insbesondere die seit langem bekannten Indolverbindungen Indican und Isatan B, die daraus erhältlichen Farbstoffe Indirubin und Indigo sowie weitere Alkaloide vom Indoltyp, Anthranilsäurederivate, Senfölglycoside und weitere Naturstoffe aus den Gruppen der Flavonoide, Terpene, Lignane, Glycoside und organische Säuren.

Erfindungsgemäss kann sowohl der praktisch nicht nachbehandelte und z.B. lediglich kontrollierte und abgepackte Gesamtextrakt verwendet werden, als auch ein solcher, der beispielsweise durch Wahl des Extraktionsmittels und/ oder der Nachbehandlung bezüglich der normalerweise im Extrakt enthaltenen Komponenten angereichert oder abgereichert worden ist, und zwar sowohl in flüssiger als auch in fester Form, und mit oder ohne die üblichen Adjuvantien, Tablettierungshilfsmittel, Löslichkeitsvermittler, Konservierungsmittel, Geschmacksstoffe und andere Zusatzstoffe, wie sie den Fachleuten der Herstellung von medizinisch verwendeten Pflanzenextrakten bekannt sind.

Bei den zur vorliegenden Erfindung führenden Untersuchungen wurde gefunden, dass Extrakte von Isatis tinctoria, die mit Extraktionsmitteln der oben genannten Art erhalten worden sind, eine signifikante Hemmung des Transkriptionsfaktors NF-κB, insbesondere eine Hemmung von mindestens etwa 50% und vorzugsweise mindestens etwa 75% ermöglichen.

Die Erfindung wird anhand der folgenden, nicht beschränkenden Beispiele weiter erläutert. Hierzu wird auch auf die beigeschlossenen Zeichnungen Bezug genommen. In den Figuren zeigen:
die Figuren 1 - 9 einige Dosis/Hemm-Kurven von in den Beispielen erhaltenen Extrakten und
die Figuren 10 - 16 Chromatogramme von in den Beispielen erhaltenen Extrakten.

### Beispiel 1

Die Wirkstoff-Isolierung erfolgte weitgehend nach der Vorschrift von Honda et al, M. Planta Medica 38 (1980) 275 - 276, hier speziell zur Gewinnung aus Isatis tinctoria L. Die Isolierung aus den anderen oben genannten Organismen kann in analoger Weise durchgeführt werden.

Ein kg getrocknetes gepulverter Pflanzenmaterial ("Droge") wurde mit jeweils 3 x 10 Liter Methanol (+2% Essigsäure) 24 Stunden erschöpfend extrahiert, der Extrakt zur Trockne eingeengt (240 g) und in Wasser (mit 1% Ammoniak) aufgenommen. Anschliessend wurde mit Dichlormethan ausgeschüttelt und der erhaltene Extrakt erneut zur Trockne eingeengt (30 g).

Der Extrakt wurde über eine Silicagelsäule stufenweise mit Hexan-Chloroform 9:1, 1:2, Chloroform und Ethylacetat zerlegt, die Chloroformfraktion mit Säulenchromatographie (Laufmittel und Chloroform) getrennt und die Tryptanthrin-Zone gewonnen. Aus der erhaltenen Fraktion fiel Tryptanthrin nach Umkristallisieren aus Ethylacetat/Chloroform in Form von gelben Kristallen aus (35 mg).

### Beispiel 2

Tryptanthrin sowie die A- und D-ringsubstituierten Indolo[2,1-b]chinazolin-6,12-dione sind nach bekannten Methoden synthetisch zugänglich, zweckmässig nach der allgemeinen Synthesevorschrift von Mitscher L. et al, Heterocycles (1981), 14, 1017-1021. Aus entsprechend substituiertem Isatin und Isatoinanhydrid (Isatoic anhydride) entsteht unter Zugabe von NaH in trockenem TMF das entsprechende Tryptanthrinderivat. Die Synthese der substituierten Isatinderivate erfolgte nach den Vorschriften von Baker und Mitscher, Patent WO95/13807. Die als Ausgangsstoffe notwendigen Anilinderivate waren kommerziell erhältlich oder nach bekannten Synthesen zugänglich (s. z.B. Fumiss et al., Practical Organic Chemistry 5. Aufl., 1989; March, J., Advanced Organic Chemistry, 3. Aufl. 1985).

Die Isatoinanhydride wurden analog den Vorschriften von Baker und Mitscher hergestellt. Als Ausgangsstoffe dienten entsprechende 2-Aminobenzoesäurederivate

### Beispiel 3

Unter Anwendung des oben beschriebenen Syntheseweges wurden u.a. folgende Indolo[2,1-b]chinazolin-6,12-dione synthetisiert:

**Tabelle 1**

| Verbindung | Substituenten R₁ bis R₈ | | | | | | | |
|---|---|---|---|---|---|---|---|---|
| Nr. | 1 | 2 | 3 | 4 | 5 | 6 | 7 | 8 |
| 1 | H | H | H | H | H | H | H | H |
| 2 | H | Cl | H | H | H | F | H | H |
| 3 | H | Cl | H | H | H | NO₂ | H | H |
| 4 | H | CH₃O | CH₃O | H | H | H | H | H |
| 5 | H | Cl | H | H | H | H | H | H |
| 6 | H | NO₂ | H | H | H | H | H | H |
| 7 | H | H | H | H | H | Br | H | H |
| 8 | H | H | H | H | H | NO₂ | H | H |
| 9 | H | H | H | H | H | F | H | H |
| 10 | H | H | H | H | H | Cl | H | H |
| 11 | H | H | H | H | H | OCH₃ | H | H |
| 12 | H | H | H | H | H | CH₃ | H | H |
| 13 | H | H | H | H | H | l | H | H |
| 14 | H | CH₃ | H | H | H | H | H | H |
| 15 | CH₃ | H | H | H | H | H | H | H |
| 16 | H | H | H | CH₃ | H | H | H | H |
| 17 | H | F | H | H | H | F | H | H |
| 18 | H | Br | H | H | H | H | H | H |
| 19 | H | F | H | H | H | H | H | H |
| 20 | H | NH₂ | H | H | H | F | H | H |
| 21 | H | H | H | H | H | H | Cl | H |
| 22 | H | H | H | H | Cl | H | H | H |
| 23 | H | H | H | CH₃O | H | F | H | H |
| 24 | H | CH₃ | H | CH₃ | H | F | H | H |
| 25 | H | CH₃ | H | H | H | F | H | H |
| 26 | H | H | F | H | H | F | H | H |
| 27 | H | H | H | H | H | H | H | F |
| 28 | F | H | H | H | H | F | H | H |
| 29 | CH₃ | H | H | H | H | F | H | H |
| 30 | H | H | H | CH₃ | H | F | H | H |
| 31 | H | H | H | H | H | F | H | F |
| 32 | H | H | NMP | H | H | F | H | H |
| 33 | H | H | H | H | NMP | H | H | H |
| 34 | H | H | H | H | H | H | NMP | H |
| 35 | NM P | H | H | H | H | F | H | H |
| 36 | H | H | H | H | H | F | NMP | H |
| 37 | H | H | H | H | H | CO₂Et | H | H |
| 38 | H | H | H | F | H | F | H | H |
| 39 | H | H | H | H | G | F | F | H |
| 40 | H | H | F | H | H | F | NMP | H |
| 41 | H | H | H | H | H | F | 3-MP | H |
| 42 | H | H | H | H | H | F | 2-MNB P | H |
| 43 | H | H | H | H | H | H | NBP | H |
| 44 | H | H | H | H | H | F | NMP | F |
| 45 | H | H | H | H | H | H | P | H |
| 46 | H | F | F | H | H | F | H | H |
| 47 | H | F | NMP | H | H | F | H | H |
| 48 | Cl | H | Cl | H | H | l | H | H |
| 49 | Cl | H | H | Cl | H | I | H | H |
| 50 | H | I | H | H | H | CO₂Et | H | H |
| 51 | H | I | H | H | H | I | H | H |
| 52 | H | H | H | OCH₃ | H | I | H | H |
| 53 | H | H | H | OCH₃ | H | H | H | H |
| 54 | H | I | H | 1 | H | I | H | H |
| 55 | H | Br | H | Br | H | I | H | H |
| 56 | H | I | H | l | H | CO₂Et | H | H |
| 57 | H | H | H | H | H | Cl | H | CH 3 |
| 58 | H | F | NBP | H | H | l | H | H |
| 59 | F | Br | H | Br | H | I | H | H |
| 60 | Cl | Br | H | Br | H | 1 | H | H |
| 61 | F | Br | H | Br | H | H | H | H |
| 62 | H | F | 3-MNBP | H | H | I | H | H |
| 63 | H | H | H | H | H | CO₂Bn | H | H |
| 64 | H | F | P | H | H | I | H | H |
| 65 | H | H | H | OH | H | I | H | H |
| 66 | H | H | H | H | H | CO₂2'Octyl | H | H |
| 67 | H | F | 3-MP | H | H | I | H | H |
| 68 | H | H | H | OCH₃ | H | CO₂Et | H | H |
| 69 | H | H | H | OCH₃ | H | SO₂n-Octyl | H | H |
| 70 | H | H | H | OCH₃ | H | SO₂NMP | H | H |
| 71 | H | H | H | OCH₃ | H | CO₂2'-Octyl | H | H |
| 72 | H | H | H | OCH₃ | H | CO₂H | H | H |
| 73 | Cl | H | Cl | H | H | FmocAG | H | H |
| 74 | Cl | H | Cl | H | H | H | H | H |
| 75 | Cl | H | Cl | H | H | AAGOCH₃ | H | H |
| 76 | H | H | H | OCH₃ | H | 1-Octenyl | H | H |
| 77 | H | F | 3-MP | H | H | CO₂H | H | H |
| 78 | H | 1-Octenyl | H | H | H | Cl | H | H |
| 79 | H | I | H | H | H | Cl | H | H |
| 80 | H | Octyl | H | H | H | Cl | H | H |
| 81 | H | OPO₃Na₂ | H | H | H | H | H | H |
| 82 | H | OH | H | H | H | H | H | H |
| 83 | H | H | F | H | H | Cl | H | H |
| 84 | H | H | H | Obn | H | F | H | H |
| 85 | H | H | NME | H | H | Cl | H | H |
| 86 | H | H | 4-M | H | H | Cl | H | H |
| 87 | H | H | Piperdin | H | H | Cl | H | H |
| 88 | H | Octyl | H | H | H | H | H | H |
| 89 | H | 6-Acoctenyl | H | H | H | Cl | H | H |
| 90 | H | NEDME | H | H | H | Cl | H | H |
| 91 | H | 6-Acoctyl | H | H | H | Cl | H | H |
| 92 | H | H | H | OH | H | F | H | H |
| 93 | H | H | DPA | H | H | Cl | H | H |
| 94 | H | H | H | OCH₃ | H | cis-1-Octenyl | H | H |
| 95 | H | 2-AG | H | H | H | Cl | H | H |
| 96 | H | H | H | OCH₃ | H | Octyl | H | H |
| 97 | H | H | H | H | H | CF₃ | H | H |
| 98 | H | H | NMEP | H | H | Cl | H | H |
| 99 | H | H | SAR | H | H | Cl | H | H |
| 100 | H | H | NME Sacch- ester | H | H | Cl | H | H |
| 101 | H | H | H | H | H | OCF₃ | H | H |
| 102 | H | H | NME Octyl-ester | H | H | Cl | H | H |
| 103 | H | H | H | H | H | n-Octyl | H | H |
| 104 | H | H | F | H | H | n-Octyl | H | H |
| 105 | H | H | n-Octyl | H | H | n-Octyl | H | H |
| 106 | H | H | NME | H | H | F | H | H |
| 107 | H | H | NME | H | H | n-Octyl | H | H |
| 108 | H | H | NMP | H | H | n-Octyl | H | H |
| 109 | H | H | F | H | H | OCF₃ | H | H |
| 110 | H | H | NME | H | H | OCF₃ | H | H |

In der obigen Tabelle 1 haben die Abkürzungen folgende Bedeutung

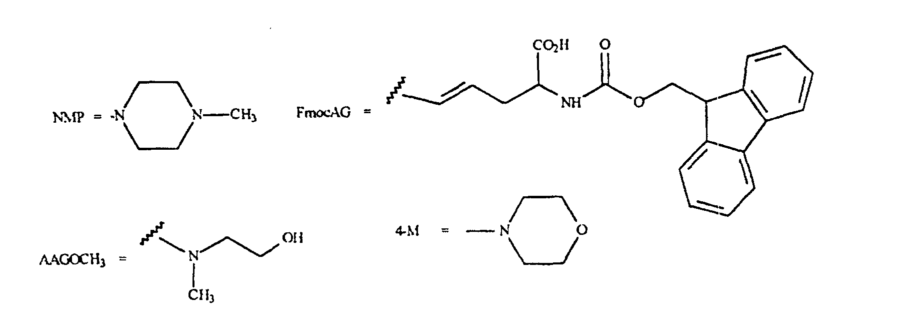

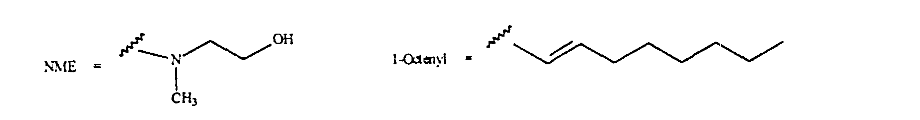

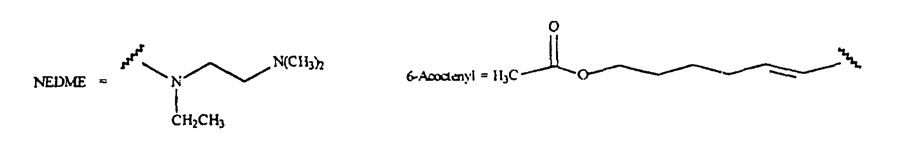

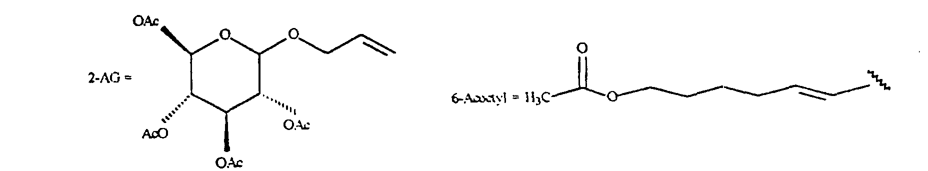

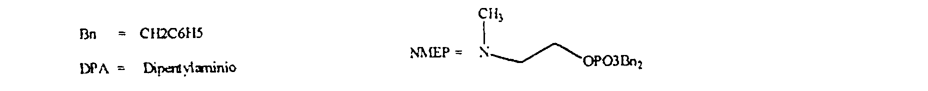

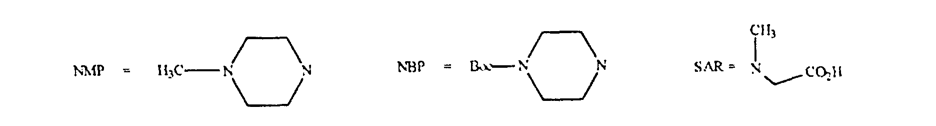

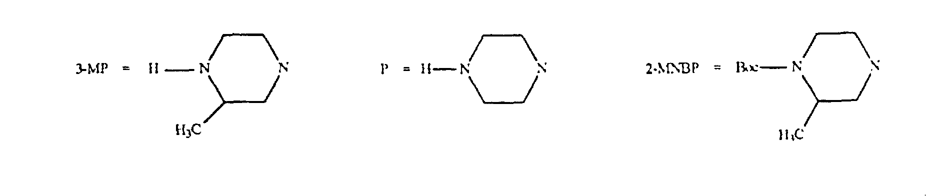

### Beispiel 4

### a. Hemmung von COX-2

Zur Bestimmung der COX-2-Hemmwirkung wurde allgemein nach der Methode von Berg et al., J. Pharmacol. Toxicol. Methods 37, 179-186 (1997) gearbeitet.

Dazu wurden Mono Mac 6 Zellen mit unterschiedlichen Konzentrationen der Testsubstanzen während 30 min inkubiert. Anschliessend wurde Lipopolysaccharid (LPS, 100 ng/ml) zupipettiert und nochmals sechs Stunden inkubiert. Anschliessend wurde Arachidonsäure zugegeben (Endkonzentration 30 µM), während 15 Min. inkubiert und das gebildete Keto-PGF1α mittels ELISA (Enzyme Linked Immuno Sorbent Assay) bestimmt.

In einer Variante wurden Testsubstanz und LPS zeitgleich eingesetzt. Die Messung der COX1 Hemmung erfolgte in HEL-Zellen nach der Methode von Berg et al., J. Pharmacol. Toxicol. Methods 37, 179-186 (1997). Dabei wurden HEL Zellen mit unterschiedlichen Konzentrationen der Testsubstanzen während 30 min inkubiert, anschliessend Arachidonsäure zugesetzt (Endkonzentration 30 µM), während 15 Min. inkubiert und das gebildete Tromboxan B2 mittels ELISA gemessen.

### b. NF-κB-Hemmung

Die gemäss Beispiel 2 hergestellten Testsubstanzen wurden in zellfreien und zellulären Testsystemen im wesentlichen nach der von Rüngeler, P. et al, Planta Medica 64, 588-593 (1998), beschriebenen Methode auf NF-κB hemmende Wirkung geprüft, verfahren. Bestimmt wurde dabei die in-vivo und die in-vitro NF-κB Bindung an DNA durch Messung der elektrophoretischen Mobilitätsverschiebung.

Die in vivo Bindung wurde mit Jurkat T-Zellen durchgeführt. Die Zellen wurden während einer Stunde mit verschiedenen Konzentrationen der verschiedenen Extrakte inkubiert und anschliessend mit TNF-α während einer Stunde stimuliert. Total-Protein-Extrakte der Zellen wurden gewonnen und in einem Assay auf elektrophoretische Mobilitätsverschiebung (electrophoretic mobility shift assay, EMSA) auf NF-κB-bindende Aktivität geprüft.

Die in vitro Bindung wurde ebenfalls mit Jurkat T-Zellen geprüft. Zellextrakle von stimulierten wie unstimulierten Zellen wurden gewonnen. Gleiche Proteinmengen (10-20 µg) wurden 2 Stunden mit Konzentrationsreihen der zu testenden Verbindungen inkubiert und schliesslich auf elektrophoretische Mobilitätsverschiebung (EMSA) analysiert. Daten ausgewählter Verbindungen sind in Tabelle 2 aufgelistet.

**Tabelle 2**

| Verbindung | COX-1 | COX-2 | NF-κB |
|---|---|---|---|
| 1 | 2 | 0,04 | 1 |
| 5 | 10 | 2 | 50 |
| 10 | 1 | 0,5 | 5 |
| 20 | 5 | 0,5 | 5 |
| 24 | >50 | >50 | >100 |
| 32 | 1 | 1 | 10 |
| 37 | >50 | >50 | >100 |
| 42 | >50 | >50 | >100 |
| 56 | )* | )* | )* |
| 60 | )* | )* | )* |
| 69 | >50 | >50 | >100 |
| 86 | 5 | 0,01 | 0,5 |
| 99 | 5 | 0,5 | 1 |
| 100 | 10 | 0,02 | 5 |
| 110 | 2 | 0,5 | 5 |
| COX-1 und COX-2: IC 50 (µM/Liter) | | | |
| NF-κB: Grenzkonzentration (µM/Liter), bei der eben noch ein | | | |
| Hemmeffekt im EMSA feststellbar ist | | | |
| )* zytotoxisch bei den geprüften Konzentrationen | | | |

Die Verbindungen 1, 10, 20, 32, 86, 99 und 110 bilden eine Gruppe bevorzugter Verbindungen, wobei die Verbindungen 1 und 86 besonders bevorzugt werden und eine besonders deutliche präferentielle Hemmung der induzierbaren COX-2 zeigen.

### Beispiele 5 - 10

Pflanzenmaterial (nachfolgend auch als "Droge" bezeichnet) von Isatis tinctoria wurde in der in Tabelle 3 angegebenen Menge mit dem ebenfalls in Tabelle 3 angegebenen Extraktionsmittel durch Bewegungsmazeration bei Raumtemperatur behandelt. Die getrocknete Droge wurde zerkleinert, dreimal während der in Tabelle 3 angegebenen Zeit extrahiert und filtriert. Die Filtrate wurden vereinigt, im Vakuum eingeengt, gefriergetrocknet und gewogen.

Bei Verwendung von frischer Droge wurde diese ohne vorherige Zerkleinerung im Turbomixer (4500 U/min) dreimal mit Lösungsmittel extrahiert und die erhaltene Aufschlämmung filtriert. Die vereinigten Filtrate wurden im Vakuum konzentriert, gefriergetrocknet und gewogen.

**Tabelle 3**

| Bsp. | Droge | Zustand | Siebsatz (mm) | Menge (g) | Lösgsm./ Menge (Liter) | Extraktionszeit (min) | Extraktmenge (g) |
|---|---|---|---|---|---|---|---|
| 5 | Blatt | trock. | 0,7 | 100 | CH₂Cl₂ 3x1 | 3x60 | 3,61 |
| 6 | Wurzel | trock. | 0,7 | 100 | CH₂Cl₂ 3x1 | 3x60 | 1,12 |
| 7 | Blatt | trock. | 0,7 | 100 | EtOH 3x1 | 3x60 | 20,71 |
| 8 | Wurzel | trock. | 0,7 | 100 | EtOH 3X1 | 3x60 | 1,28 |
| 9 | Blatt | frisch | - | 100 | EtOH 3x0,75 | 3x60 | 3,81 |
| 10 | Wurzel | frisch | - | 100 | EtOH 3X0,75 | 3x60 | 2,07 |

### Beispiele 11 - 15

Die trockene Droge wurde zerkleinert (Siebsatz 0,7mm) und unter Laborbedingungen während den in Tabelle 4 angegebenen Zeiten sukzessive mit überkritischem CO₂, CO₂/Ethanol (Ethanolanteil 20 Vol.%) als "Fluid" bei 50 bzw. 90°C unter den ebenfalls angegebenen Verfahrensparametern (Extraktionsdauer = Zeit, Gasfluss = Fluss, Temperatur = Temp., Extraktmenge = Extr.) extrahiert. Der Extrakt wurde gegebenenfalls im Vakuum eingeengt und gefriergetrocknet.

**Tabelle 4**

| B. | Droge | Menge (g) | Fluid | Zeit (min) | Fluss (ml/min) | Tmp. (°C) | Druck (bar) | Extr. (mg) |
|---|---|---|---|---|---|---|---|---|
| 11 | Blatt | 18 | CO₂ | 360 | 210-290 | 50 | 300 | 264 |
| 12 | Blatt | 18 | 20%)* | 300 | 200-290 | 50 | 300 | 878 |
| 13 | Blatt | 18 | 20%)* | 300 | 200-290 | 90 | 300 | 151 |
| 14 | Blatt | 18 | CO₂ | 420 | 200-290 | 50 | 300 | 307 |
| 15 | Blatt | 18 | 20%)* | 300 | 200-290 | 50 | 300 | 993 |

| | | | | | | | | |
|---|---|---|---|---|---|---|---|---|
| )* jeweils eine Mischung aus CO₂ und 20% EtOH | | | | | | | | |

Die HPLC-Analyse der Extrakte der Beispiele 5 - 15 ergab ferner, dass sich die Zusammensetzungen nicht wesentlich unterschieden. Die DC-Analyse zeigte ferner eine weitgehende Übereinstimmung des Extrakts von Beispiel 11 und Hexanextrakt.

### Beispiele 16 - 21

Bei diesen Beispielen wurde ähnlich wie in den Beispielen 11-15 gearbeitet, jedoch unter Verwendung einer SFE-Laboranlage ISCO 2100, die mit zwei 100DX Hochdruckspritzpumpen für CO₂ und Modifier ausgerüstet war, und mit den in der folgenden Tabelle 5 angegebenen Materialien, Mengen, Betriebs- und Ergebnisdaten.

**Tabelle 5**

| Beisp | Droge | Menge (g) | Fluid | Zeit (min) | Fluss (ml/min) | Temp. (°C) | Druck (bar) | Extr. (mg) |
|---|---|---|---|---|---|---|---|---|
| 16 | Blatt | 18 | CO₂ | 180 | 0,8-1,0 | 50 | 500 | 68 |
| 17 | Blatt | 18 | CO₂ | 180 | 0,8-1,0 | 50 | 690 | 144 |
| 18 | Blatt | 18 | CO₂* | 180 | 0,8-1,0 | 50 | 350 | 173 |
| 19 | Blatt | 18 | CO₂** | 180 | 0,8-1,0 | 50 | 350 | 55 |
| 20 | Blatt | 18 | CO₂*** | 180 | 0,8-1,0 | 50 | 350 | 253 |
| 21 | Blatt | 18 | CO₂*** | 180 | 0,8-1,0 | 50 | 690 | 313 |

| | | | | | | | | |
|---|---|---|---|---|---|---|---|---|
| CO₂* = CO₂ mit 0,5 Vol.% EtOH-H₂O (1:1 Vol.) | | | | | | | | |
| CO₂** = CO₂ mit 0,5 Vol.% H₂O | | | | | | | | |
| CO₂*** = CO₂ mit 0,5 Vol.% EtOH | | | | | | | | |

Wie Tabelle 5 zeigt, ist die Hochdruckextraktion mit CO₂ in Mischung mit bei Normalbedingungen flüssigem Lösungsmittel eine bevorzugte Form der Herstellung eines erfindungsgemäss zu verwendenden Extraktes.

### Beispiel 22

Zur Untersuchung des pharmakologischen Profils in vitro wurden Extrakte, die gemäss den Beispielen 5, 6, 7, 11 und 12 hergestellt waren, in zellfreien und zellulären Testsystemen nach bekannten Methoden auf NF-κB hemmende Wirkung geprüft. Dabei wurde im wesentlichen nach den von Rüngeler, P. et al., Planta Medica 64, 588-593 (1998), beschriebenen Methoden verfahren. Bestimmt wurden die in vivo und in vitro NF-κB Bindung an DNA, durch Bestimmung der elektrophoretischen Mobilitäts-Verschiebung.

Die Bindung in vivo wurde mit Jurkat T-Zellen durchgeführt. Die Zellen wurden mit verschiedenen Konzentrationen der verschiedenen Extrakte während einer Stunde inkubiert und anschliessend mit TNF-α während einer Stunde stimuliert.

Totalprotein-Extrakte der Zellen wurden gewonnen und auf NF-κB-bindende Aktivität in einem Assay auf elektrophoretische Mobilitäts-Verschiebung (electrophoretic mobility shift assay, EMSA) geprüft.

Die Bindung in vitro wurde ebenfalls mit Jurkat T-Zellen geprüft. Es wurden Zellextrakte von stimulierten und von unstimulierten Zellen wurden gewonnen. Gleiche Proteinmengen (10-20 µg) wurden nach für 2 Stunden Inkubation mit Konzentrationsreihen der verschiedenen Isatis-Extrakte und zum Schluss durch EMSA analysiert.

**Tabelle 6**

| Hemmung der Aktivierung von NF-κB in Jurkat T- Zellen | | | | | |
|---|---|---|---|---|---|
| Konz. (µg/ml) | 5 | 6 | 7 | 11 | 12 |
| 100 | + | + | + | + | + |
| 50 | + | + | + | + | + |
| 25 | (+) | (+) | - | - | (+) |
| 10 | - | - | - | - | - |
| + : Hemmung der Aktivierung von NF-κB; Reduzierung der Aktivierung; | | | | | |
| - : keine Hemmung | | | | | |

### Beispiel 23

Die in den Beispielen 15, 17 und 20 hergestellten Extrakte wurden auf ihre dosisabhängige Hemmung von COX-2, Histamin- und Serotonin-Freisetzung geprüft. Die Prüfungen der COX-2-inhibitorischen Wirkung wurde gemäss der in Beispiel 4 beschriebenen Arbeitsweise geprüft.

Die Hemmung der Histamin-Freisetzung wurde nach der Arbeitsweise von Hakanson et. al., Anal. Biochem 47 (1972) 356-370 an Ratten-Mastzellen nach Stimulation mit der dort als Compound 48/80 bezeichneten Substanz.

Die Hemmung der Serotonin-Freisetzung wurde nach der Arbeitsweise von Theoharides et al., Biochem. Pharmacol 34 (1985) 1389-1398, ebenfalls an Ratten-Mastzellen bestimmt.

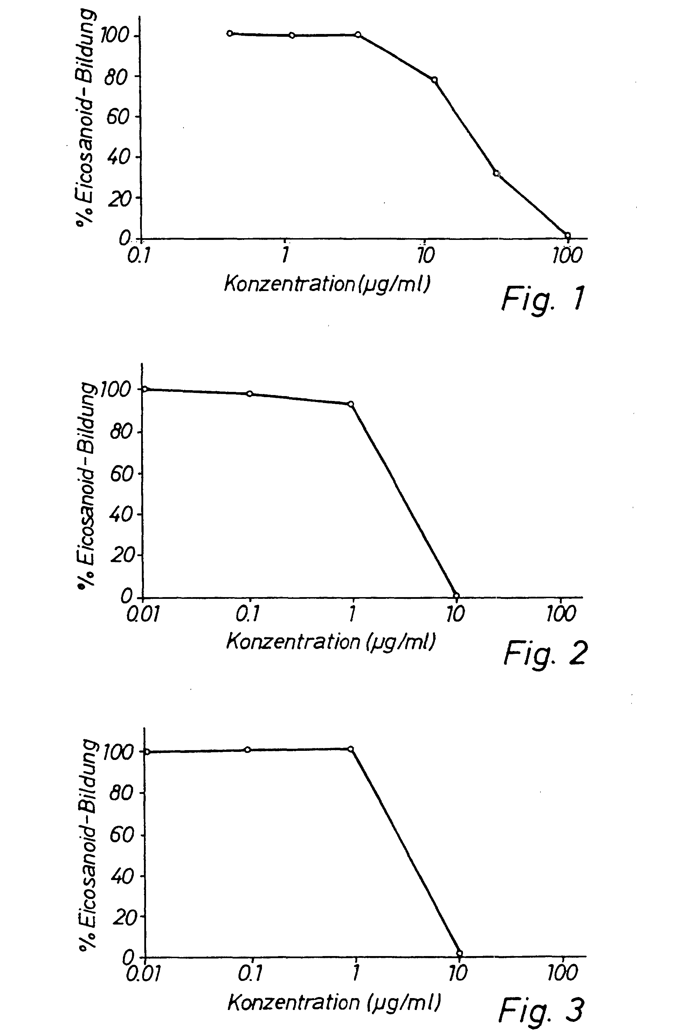

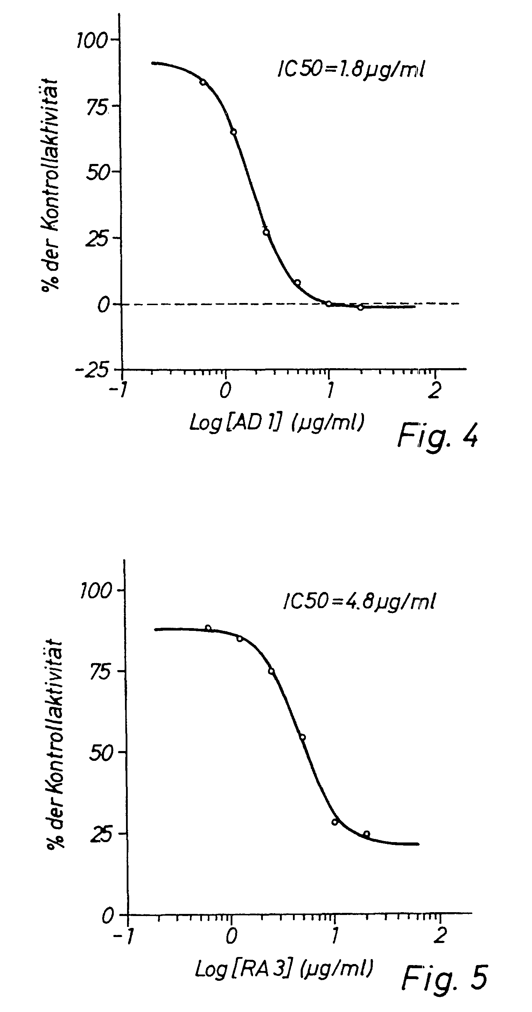

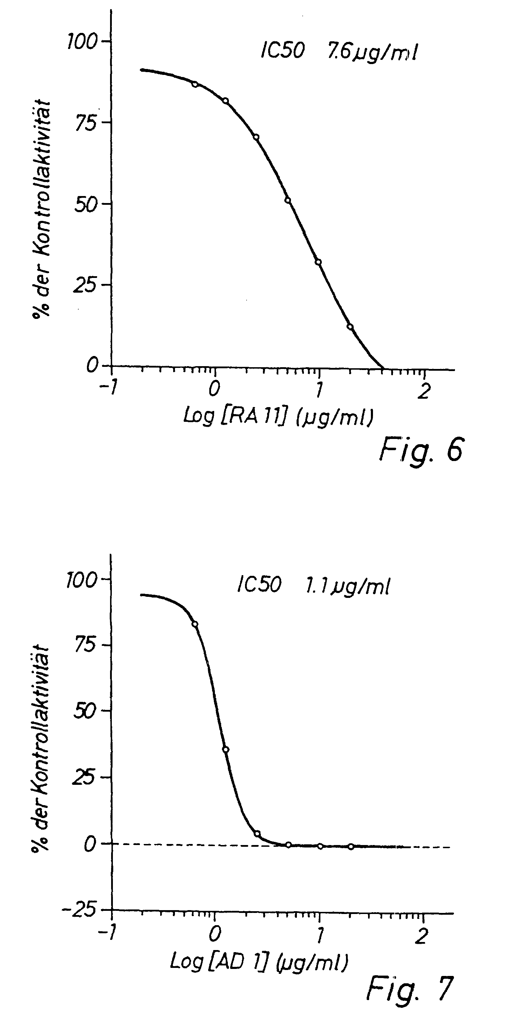

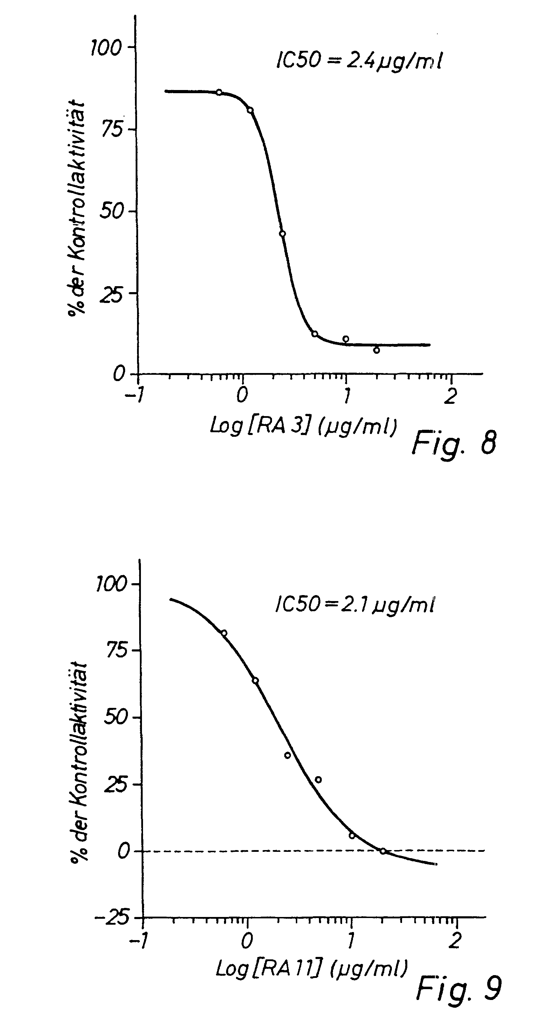

Die so erhaltenen Dosis-Hemmkurven sind in den Figuren 1 - 9 dargestellt. Die Berechnung der IC₅₀-Werte lieferte folgende Resultate:

**Tabelle 7**

| Beispiel | COX-2 | Histamin-Freisetzung | Serotonin-Freisetzung |
|---|---|---|---|
| 15 | 30 | 1,8 | 1,1 |
| 17 | 4,9 | 4,8 | 2,4 |
| 20 | 5,1 | 7,6 | 2,1 |

### Beispiel 24

Die Extrakte wurden jeweils durch ihre Chromatogramm (chromatographischer Fingerprint charakterisiert, die mit Hilfe der Hochdruck-Flüssigchromatographie (HPLC) aufgenommen wurden. Hierzu wurde eine binäre Hochdruckgradienten-Anlage (Hewlett-Packard HP1100) mit Diodenarray-Detektor, Probengeber und Säulenofen verwendet. Die Trennbedingungen waren wie folgt:
Trennsäule: LiChroCART Kartusche (4 x 125 mm), gepackt mit LiChrospher 100 RP-18 von 5µm mittlerer Partikelgrösse;
Lösungsmittelprogramm: H₂O-Acetonitril 90:10 für 5 min, dann H₂O-Acetonitril-(iradient von 90:10 nach 10:90 in 45 min, H₂O-Acetonitril 10:90 für 15 min, anschliessend H₂O-Acetonitril-Gradient von 10:90 nach 90:10 in 15 min;
Flussrate: 1 ml/min; Detektion bei 254nm und 387 nm.

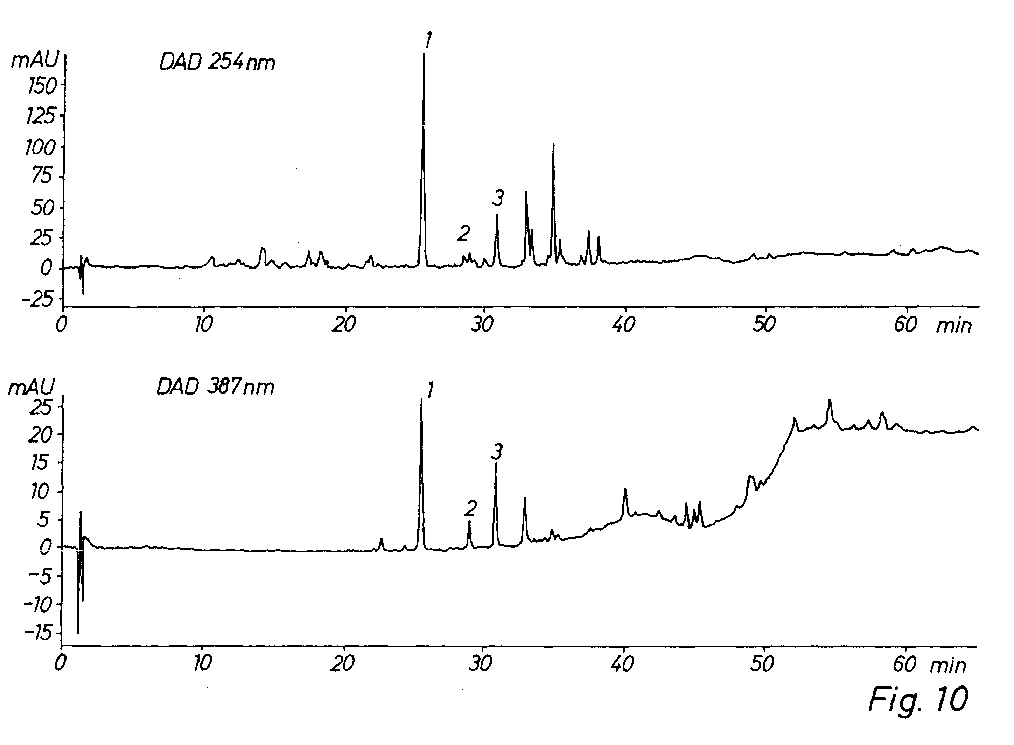

Das Chromatogramm des Extraktes von Beispiel 14 ist in Fig. 10 abgebildet, mit Detektion bei 254 nm (oben) und 387 nm (unten). Die für Isatis typischen und bekannten Stoffe, die als Leitsubstanzen für die Charakterisierung der Extrakte beigezogen werden können, sind Tryptantrin (1), Indigo (2) und Indirubin (3) Diese Substanzen konnten durch ihre charakteristischen UV-vis Spektren und durch Vergleich mit Referenzsubstanzen eindeutig identifiziert werden.

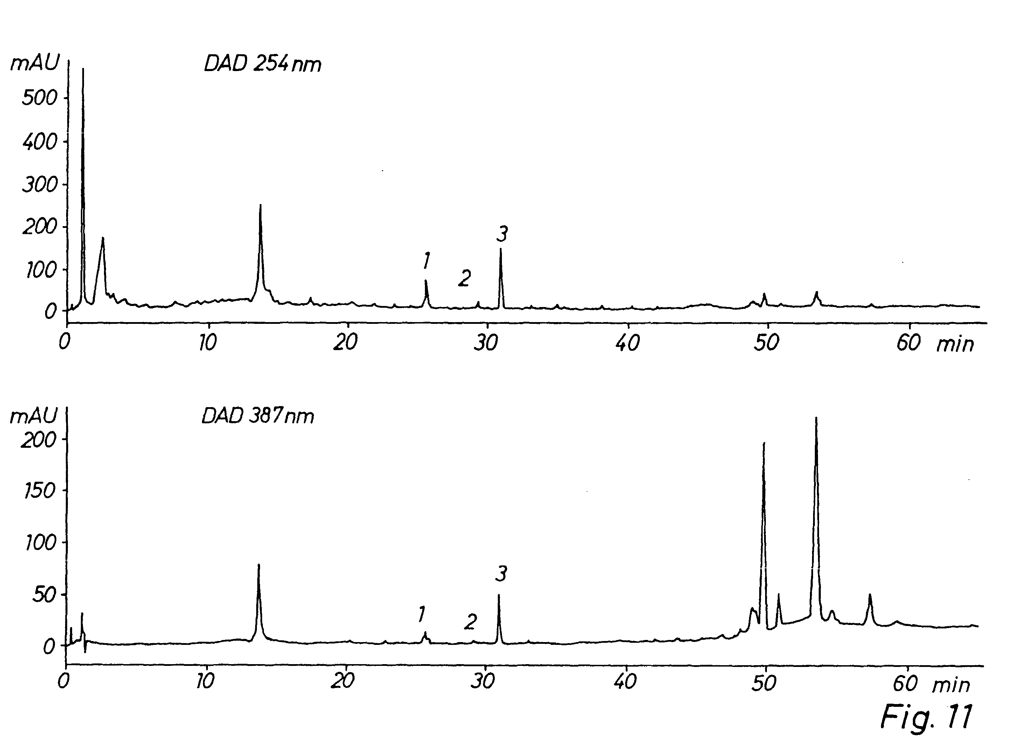

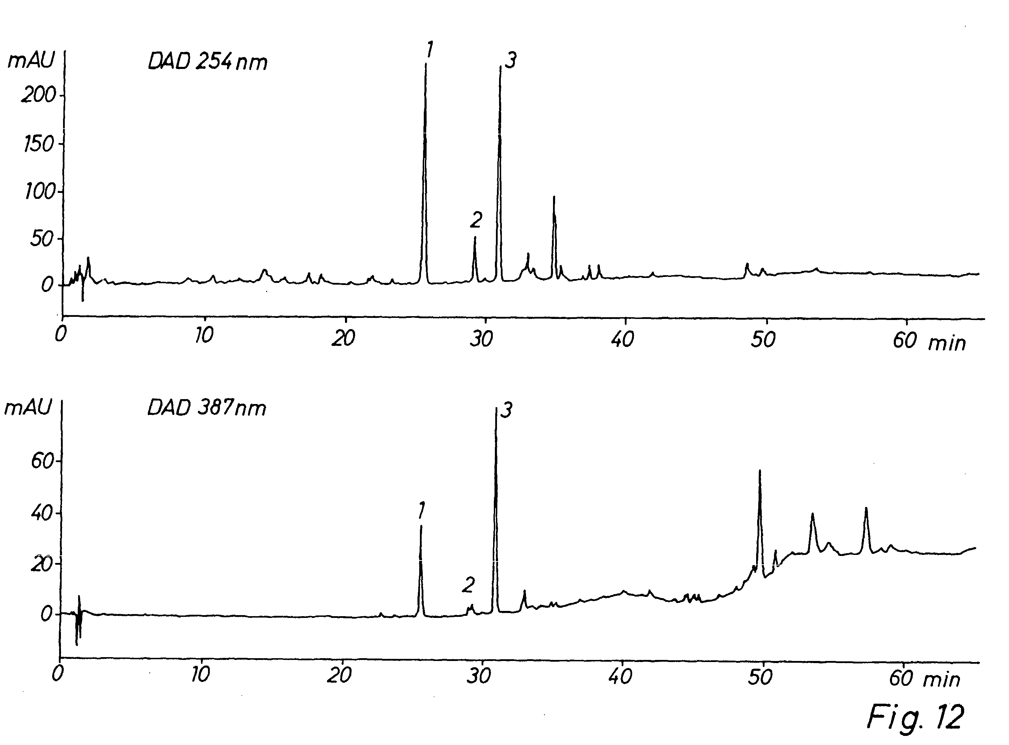

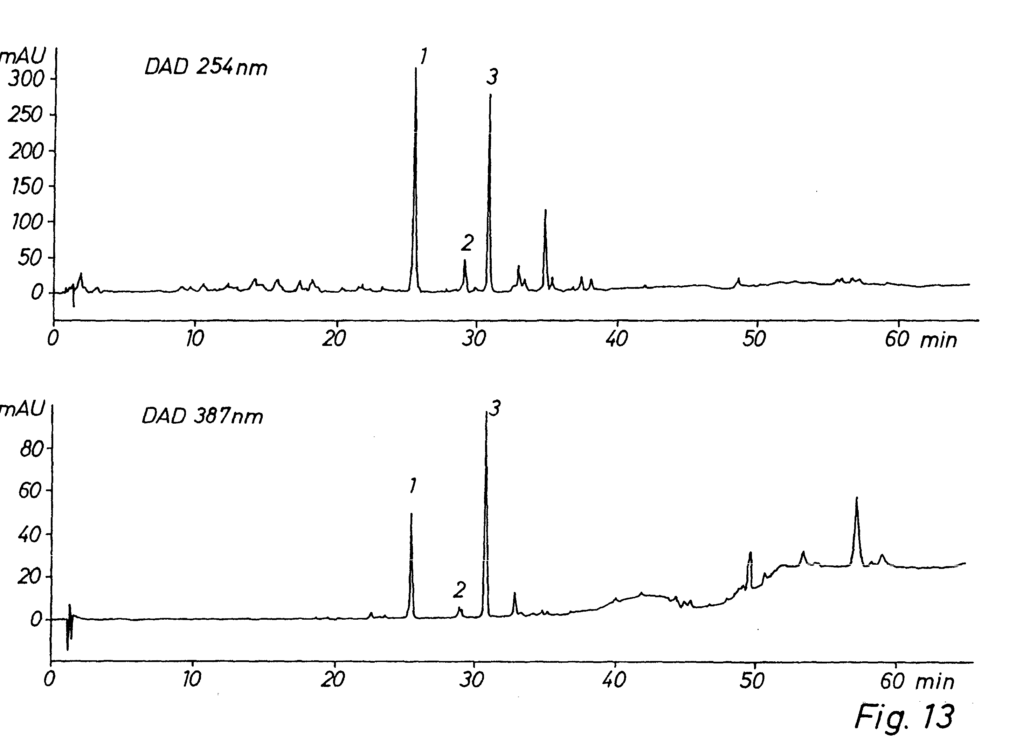

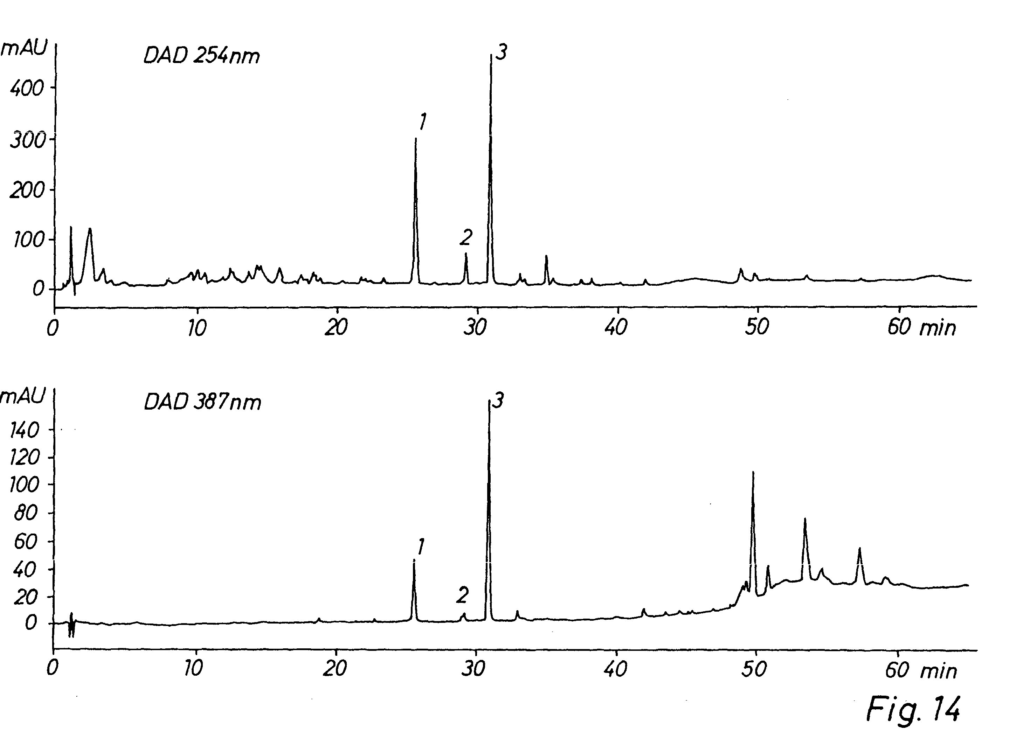

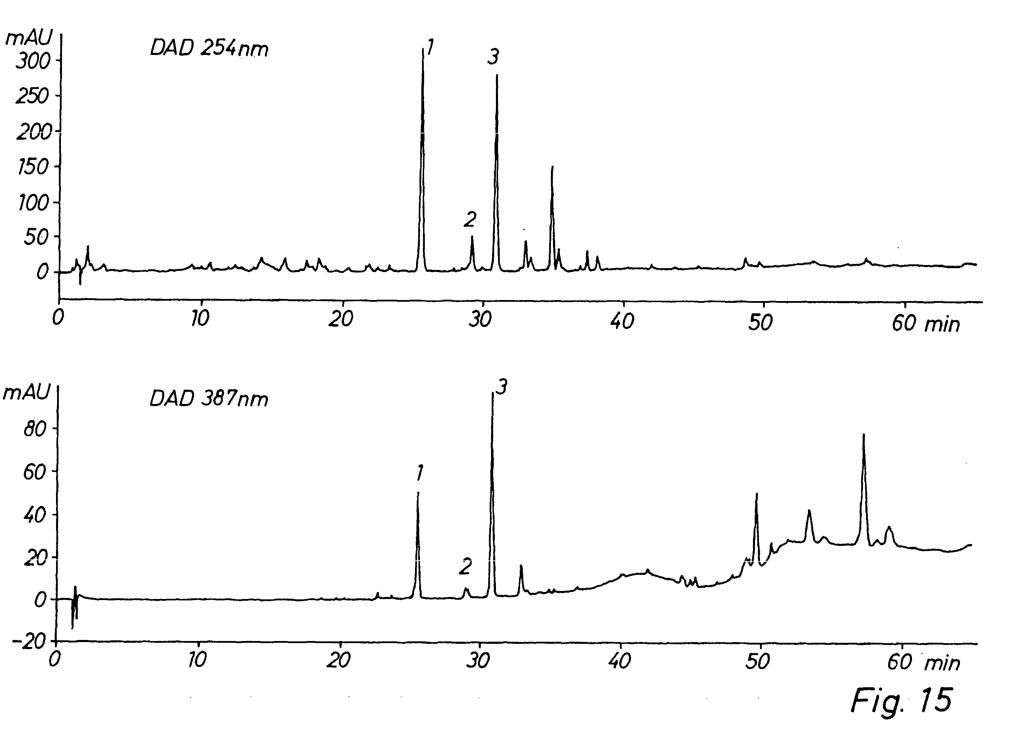

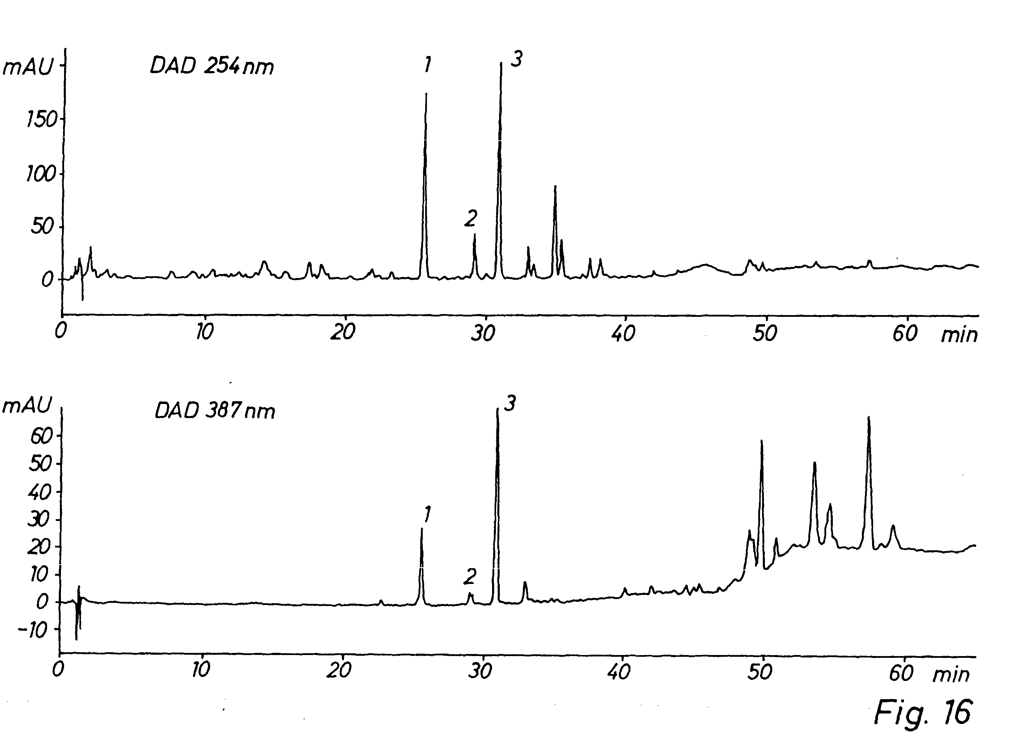

Fig. 11 zeigt das Chromatogramm des Extraktes von Beispiel 15, Fig. 12 den Fingerprint des Extraktes nach Beispiel 17 und Fig. 13 einen Extrakt nach Beispiel 18. Die Trennung eines Extraktes, der gemäss Beispiel 19 gewonnen wurde, ist in Fig. 14 wiedergeben. Ein Extrakt nach Beispiel 20 ist in Fig. 15 abgebildet und ein Extrakt nach Beispiel 21 in Fig. 16. In allen SFE-Extrakten sind die Verbindungen (1), (2) und (3) enthalten.

Die Dosierung von erfindungsgemäss verwendbaren Isatis-Extrakten, die durch die oben erläuterten Chromatogramme charakterisieren lassen, liegt allgemein in dem für vergleichbare Pflanzenextrakte üblichen Bereich von 50 mg bis 1000 mg/Tag. Eine kritische toxizitätsbedingte Obergrenze konnte bisher nicht ermittelt werden. Dabei können die Extrakte erfindungsgemäss als solche oder in Mischung mit den zur Herstellung fester oder flüssiger Arzneimittelzubereitungen bekannten Zusatz- oder Hilfsstoffen und/oder kombiniert mit anderen therapeutisch wirksamen Substanzen verwendet werden.

Die beschriebenen Extrakte können erfindungsgemäss als solche oder in Mischung mit den zur Herstellung fester oder flüssiger Arzneimittelzubereitungen bekannten Zusatz- oder Hilfsstoffen und/oder mit anderen therapeutisch wirksamen Substanzen verwendet werden.

Zusammenfassend bietet die Erfindung neue Mittel, die sich zur Behandlung von Leiden eignen, die mit NF-κB in direktem oder indirektem Zusammenhang stehen, insbesondere als antiphlogistische bzw. antirheumatische Mittel anstelle oder zusammen mit bekannten antiphlogistischen Wirkstoffen, zur präventiven oder adjuvanten Behandlung von neurodegenerativen Erkrankungen, von Morbus Alzheimer und von Krebserkrankungen sowie zur präventiven Dauermedikation, insbesondere gegen Altersbeschwerden.

## Patentansprüche

1. Verwendung von Verbindungen der Formel (I) 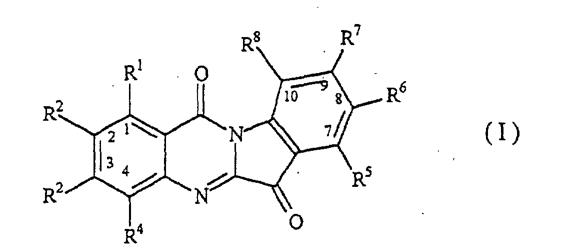 in der R¹ bis und mit R⁸ unabhängig voneinander gewählt sind aus Wasserstoffatomen und pharmazeutisch akzeptablen Substituenten, sowie pharmazeutisch akzeptable Salze von Verbindungen der Formel (I) und Stoffmischungen, die mindestens eine Verbindung der Formel (I) oder ihr pharmazeutisch akzeptables Salz enthalten, zur Herstellung von Arzneimitteln zur Hemmung des Transkriptionsfaktors NF-κB und/oder zur Hemmung von COX mit einer präferentiellen Hemmwirkung auf COX-2.

2. Verwendung nach Anspruch 1 für die Herstellung von Arzneimitteln mit antiphlogistischer, insbesondere antirheumatischer Wirkung zur Behandlung von entzündlichen Erkrankungen, insbesondere des rheumatischen Formenkreises und der Arthritiden.

3. Verwendung nach Anspruch 1 für die Herstellung von Arzneimitteln zur präventiven oder adjuvanten Behandlung von Morbus Alzheimer.

4. Verwendung nach einem der Ansprüche 1 - 3, **dadurch gekennzeichnet, dass** als Stoffmischung, die mindestens eine Verbindung der Formel (I) enthält, ein durch Extraktion von Isatis tinctoria mit einem mindestens teilweise aus CO₂ bestehenden Extraktionsmittel erhaltener Extrakt verwendet wird.

## Claims

1. A use of compounds of formula (I) 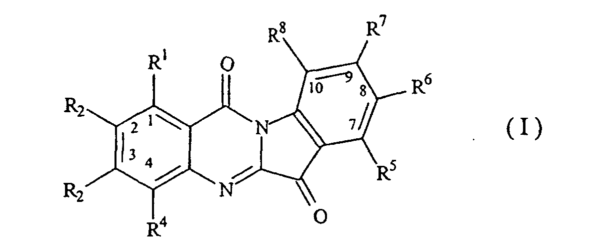 wherein R¹ through R⁸ are selected independently from hydrogen atoms and pharmaceutically acceptable substituents, as well as of pharmaceutically acceptable salts of compounds of formula (I) and of mixtures of substances containing at least one compound of formula (I) or a pharmaceutically acceptable salt thereof for preparation of medicaments for inhibiting transcription factor NF-κB and/or for inhibiting COX with a preferential inhibitory effect upon COX-2.

2. The use of claim 1 for preparation of medicaments having an anti-phlogistic and notably an anti-rheumatic effect for treatment of inflammatory diseases, notably in the area of rheumatic disease forms.

3. The use of claim 1 for preparation of medicaments for preventive or adjuvant treatment of Morbus Alzheimer.

4. The use according to any of claims 1 - 4, **characterized in that** an extract obtained by extraction of Isatis tinctoria with an extracting agent consisting at least in part of CO₂ is used as said mixture of substances containing at least one compound of formula (I).

## Revendications

1. Utilisation de composés de la formule (1) 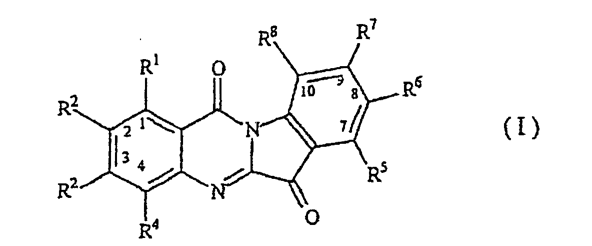 dans laquelle R¹ à R⁸ inclus, sont choisis indépendamment les uns des autres parmi les atomes d'hydrogène et des substituants pharmaceutiquement acceptables ainsi que des sels pharmaceutiquement acceptables, de composés de la formule (I) et de mélanges de substances, qui contiennent au moins un composé de la formule (I) ou son sel pharmaceutiquement acceptable, en vue de la fabrication de médicaments destinés à l'inhibition du facteur de transcription NF-κB et/ou à l'inhibition de COX avec une action inhibitrice préférentielle sur COX-2.

2. Utilisation selon la revendication 1, en vue de la fabrication de médicaments à action antiphlogistique, en particulier à action antirhumatismale, destinés au traitement de maladies inflammatoires, en particulier des troubles rhumatismaux et des arthrites.

3. Utilisation selon la revendication 1, en vue de la fabrication de médicaments destinés au traitement préventif ou adjuvant de la maladie d'Alzheimer

4. Utilisation selon l'une quelconque des revendications 1-3, **caractérisé en ce que** l'on utilise, en tant que mélange de substances, qui contient un composé de la formule (I), un extrait obtenu par extraction d'Isatis tinctoria à l'aide d'un agent d'extraction se composant an moins partiellement de CO₂.
